(19) Europäisches Patentamt
European Patent Office
Office européen des brevets

(11) Veröffentlichungsnummer: **0 413 200 B1**

(12) # EUROPÄISCHE PATENTSCHRIFT

(45) Veröffentlichungstag der Patentschrift: **05.10.94**

(51) Int. Cl.5: **C07C 309/26**, C07C 309/50, C07C 309/52, C07C 303/28, G03F 7/022

(21) Anmeldenummer: **90114801.5**

(22) Anmeldetag: **02.08.90**

(54) **Substituierte 1,2-Naphthochinon-(2)-diazid-4-sulfonsäuren, Verfahren zu ihrer Herstellung und ihre Verwendung.**

(30) Priorität: **12.08.89 DE 3926776**

(43) Veröffentlichungstag der Anmeldung:
**20.02.91 Patentblatt 91/08**

(45) Bekanntmachung des Hinweises auf die Patenterteilung:
**05.10.94 Patentblatt 94/40**

(84) Benannte Vertragsstaaten:
**BE CH DE FR GB LI**

(56) Entgegenhaltungen:
**EP-A- 0 198 800**
**EP-A- 0 212 482**
**EP-A- 0 283 898**
**EP-A- 0 368 085**
**DD-A- 263 982**
**DE-A- 3 837 500**
**DE-C- 1 126 541**
**US-A- 4 576 901**

(73) Patentinhaber: **HOECHST AKTIENGESELL-SCHAFT**

**D-65926 Frankfurt (DE)**

(72) Erfinder: **Scheler, Siegfried, Dr. Dipl.-Chem.**
**Stormstrasse 5**
**D-6200 Wiesbaden-Naurod (DE)**
Erfinder: **Buhr, Gerhard, Dr. Dipl.-Chem.**
**Am Erdbeerstein 28**
**D-6240 Königstein (DE)**
Erfinder: **Bergmann, Klaus**
**Buchenweg 57**
**D-6500 Mainz-Bretzenheim (DE)**

EP 0 413 200 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft substituierte 1,2-Naphthochinon-(2)-diazid-4-sulfonsäuren, Verfahren zu ihrer Herstellung und ihre Verwendung.

Die erfindungsgemäßen Verbindungen besitzen die allgemeine Formel A

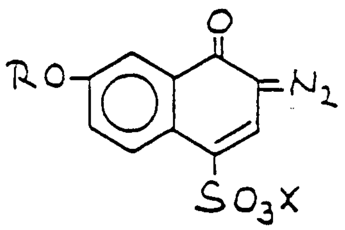

A

in der

R eine Epoxyalkyl-, Alkylcarbonyl oder Alkylsulfonylgruppe, deren Kohlenstoffketten durch Sauerstoffatome unterbrochen sein können, eine gegebenenfalls substituierte Aralkyl-, Arylcarbonyl- oder Arylsulfonylgruppe

und

X Wasserstoff, Metall oder eine Ammoniumgruppe bedeuten.

Vorzugsweise bedeuten

R eine Epoxyalkylgruppe mit 3 bis 6 Kohlenstoffatomen, eine Alkylcarbonylgruppe mit 2 bis 6 Kohlenstoffatomen oder eine Alkylsulfonylgruppe mit 1 bis 4 Kohlenstoffatomen, deren Kohlenstoffketten durch wenigstens ein Sauerstoffatom unterbrochen sein können,
eine Aralkylgruppe mit 7 bis 10 Kohlenstoffatomen, eine Arylcarbonylgruppe mit 7 bis 10 Kohlenstoffatomen, eine Arylsulfonylgruppe mit 6 bis 10 Kohlenstoffatomen, die durch Alkyl-, Trihalogenalkyl-, Alkoxy-, Alkylcarbonyl-, Alkylsulfonylgruppen oder Halogen substituiert sein können, und

X Alkali- oder Erdalkalimetall, z.B. Kalium oder Natrium, oder die Ammoniumgruppe

bedeuten.

Ester, Amide und Hydrazide der 1,2-Naphthochinon-(2)-diazidsulfonsäuren werden schon seit vielen Jahren als lichtempfindliche Verbindungen für strahlungsempfindliche Gemische, wie etwa Photoresists für die Herstellung von Halbleiterbauelementen in der Mikroelektronik oder als Beschichtungslösungen für die Herstellung von photomechanisch verarbeitbaren Druckformen oder Farbprüffolien eingesetzt. Geeignete Verbindungen und Verarbeitungsverfahren sind beschrieben in: J. Kosar, "Light Sensitive Systems", John Wiley & Sons, New York, Kap. 7.4, 1965, US-PS 4 104 070, US-PS 4 576 901 und EP-A 0 212 482.

Die Herstellung der diesen bekannten Derivaten zugrunde liegenden 1,2-Naphthochinon-(2)-diazidsulfonsäuren geht aus von der 1-Naphthol-4- bzw. -5-sulfonsäure, die mit Alkalinitrit in verdünnter Mineralsäure nitrosiert wird. Die entstandenen 2-Nitroso-1-naphtholsulfonsäuren werden isoliert, und die nicht umgesetzten Ausgangsstoffe sowie die bei der Nitrosierung entstandenen Nebenprodukte durch Auswaschen oder Umlösen entfernt. Die Nitrosoverbindung wird anschließend in wäßriger Lösung zur entsprechenden Aminoverbindung reduziert. Diese wird isoliert und erneut von nicht umgesetzten Ausgangsstoffen und entstandenen Nebenprodukten durch Digerieren in Wasser oder durch Umlösen befreit. Anschließend wird die Aminoverbindung in Wasser suspendiert und bei einem pH-Wert von 4 - 6 mit Alkalinitrit in Anwesenheit von Cu (II)-Salzen diazotiert. In den meisten Fällen müssen die auf diese Weise gewonnenen 1,2-Naphthochinon-(2)-diazidsulfonsäuren noch durch Umlösen oder Umkristallisieren von den bei der Diazotierung entstandenen dunkel gefärbten Nebenprodukten befreit werden.

Der Nachteil dieses bekannten Herstellungsverfahrens besteht im wesentlichen darin, daß die bei den einzelnen Reaktionsstufen nicht umgesetzten Einsatzstoffe und die entstandenen Nebenprodukte vom gewünschten Hauptprodukt durch zusätzliche Reinigungsschritte abgetrennt werden müssen. Damit verbunden sind niedrige Ausbeuten, eine nicht immer befriedigende Produktqualität und hohe Herstellungskosten.

Aus der EP-A 0 283 898 ist ein Verfahren zur Herstellung von gegebenenfalls durch Halogen, Nitro- oder Alkylgruppen substituierten Benzo- und Naphthochinon-diazid-sulfonsäuren und deren Salzen bekannt. Man geht hierbei aus von einer Arylsulfonsäure mit mindestens einer Hydroxygruppe, nitrosiert in bekannter Weise, reduziert die entstandene Nitrosoverbindung im alkalischen pH-Bereich und wandelt anschließend die Aminoverbindung in ein Sulfamatderivat um, das anschließend mit einem Diazotierungsagenz gemischt wird. Nach dem Ansäuern der Mischung erhält man die Benzo- bzw. Naphthochinon-diazid-sulfonsäuren.

Die nach jedem Reaktionsschritt entstandenen Reaktionsprodukte werden nicht zwischenisoliert, sondern verbleiben zur Weiterreaktion in Lösung ("Eintropfreaktion"). Nebenprodukte und Verunreinigungen, die bei den einzelnen Verfahrensschritten anfallen, können durch Filtration der Reaktionslösung in befriedigender Weise abgetrennt werden.

Nachteilig an diesem Einstufenverfahren ist, daß die verschiedenen Verfahrensschritte nur in einem relativ kleinen pH-Bereich durchführbar sind und die Reaktionszeiten und Reaktionstemperaturen sehr exakt eingehalten werden müssen. Die nach diesem Verfahren erhaltenen Endprodukte sind in der Regel nicht frei von isomeren Verbindungen. Deshalb ist ein universeller technischer Einsatz der nach diesem Verfahren hergestellten Verbindungen eingeschränkt.

In der nicht vorveröffentlichten deutschen Patentanmeldung, Aktenzeichen P 38 37 499.4 wird ein Verfahren zur Herstellung von Estern kernsubstituierter 1,2-Naphthochinon-(2)-diazid-4-sulfonsäuren beschrieben, bei dem als Zwischenstufen 1,2-Naphthochinon-(2)-diazid-4-sulfonsäuren anfallen, die in 5-, 6-, 7- oder 8-Position durch Halogen, Alkoxy oder Alkoxycarbonyl substituiert sind.

Man geht hierbei aus von einem entsprechend substituierten 2-Naphthol, nitrosiert in 1-Position, sulfoniert mit Alkalihydrogensulfit in 4-Position und reduziert anschließend die Nitrosogruppe durch Ansäuern mit Mineralsäure bei einem pH-Wert < 7 zur Aminogruppe, oxidiert die 2-Amino-1-naphthol-4-sulfonsäure zur entsprechenden 1,2-Naphthochinon-4-sulfonsäure und setzt diese mit p-Toluolsulfonsäurehydrazid in einem organischen Lösungsmittel bei Temperaturen von 20 - 100 °C zu der entsprechenden kernsubstituierten 1,2-Naphthochinon-(2)-diazid-4-sulfonsäure um.

Durch Chlorierung mit Chlorsulfonsäure bzw. einem Gemisch aus Chlorsulfonsäure/Thionylchlorid erhält man in bekannter Weise das Sulfonsäurechlorid, das anschließend mit einer phenolischen Komponente zu den entsprechenden kernsubstituierten 1,2-Naphthochinon-(2)-diazid-4-sulfonsäureestern kondensiert wird. Die einzelnen Verfahrensschritte zur Herstellung der Zwischen- und Endprodukte sind aus der Literatur bekannt. Das Verfahren ist abhängig von der relativ schweren Zugänglichkeit der als Ausgangsstoffe verwendeten substituierten 2-Naphthole.

So erhält man beispielsweise 7-Alkoxy-2-naphthol nach bekanntem Verfahren durch Monoalkylierung von 2,7-Dihydroxynaphthalin in einer Ausbeute von nur etwa 50 - 55 % d. Theorie. Die Ausbeuten der anschließenden Reaktionsstufen - Nitrosierung, Sulfonierung und Reduktion, Oxidation, Einführung der Diazogruppe - sind zufriedenstellend. Trotz Verzicht auf zusätzliche Reinigung der Zwischenstufen ist die Gesamtausbeute an 7-Alkoxy-1,2-napthochinon-(2)-diazid-4-sulfonsäure, bezogen auf das eingesetzte 2,7-Dihydroxynaphthalin, noch nicht zufriedenstellend, so daß die Produktionskosten für die herstellbaren Sulfonsäureester relativ hoch sind.

Ein weiteres Verfahren zur Herstellung kernsubstituierter 1,2-Naphthochinon(2)-diazid-4-sulfonsäuren, die zur Synthese der entsprechenden Ester und Amide verwendet werden können, wird in der nicht vorveröffentlichten deutschen Patentanmeldung, Aktenzeichen P 38 37 500.1, angegeben. Zur Herstellung von 7-Methoxy1,2-naphthochinon-(2)-diazid-4-sulfonsäure, geht man beispielsweise von käuflichem 1-Acetylamino-7-naphthol aus. Dieses kann in einer 7-stufigen Reaktionsfolge - Methylierung der phenolischen Hydroxylgruppe, Abspaltung der Acetylgruppe und Herstellung von 1-Amino-7-methoxynaphthalin-Hydrogensulfat, Sulfonierung in 4-Position durch trockenes Erhitzen ("Backreaktion"), Austausch der Aminogruppe durch eine Hydroxylgruppe ("Bucherer Reaktion"), Nitrosierung in 2-Position, Reduktion der Nitroso zur Aminogruppe, Diazotierung - zu der gewünschten Verbindung umgesetzt werden.

Wegen der Mehrstufigkeit und wegen betriebstechnisch schwierig durchführbarer Verfahrensschritte sowie der nicht immer zufriedenstellenden Ausbeute einzelner Zwischenstufen ist auch dieses Herstellungsverfahren technisch problematisch. Für die Herstellung von Photolacken ist es jedoch von größter Wichtigkeit, über ein wirtschaftliches Syntheseverfahren der strahlungsempfindlichen Komponenten zu verfügen.

Aus DD-A 263 982 ist die Herstellung von 2-Diazo-1-oxo-1,2-dihydronaphthalinderivaten, ausgehend von 1,7-Dihydroxynaphthalinderivaten bekannt. Die hierzu als Ausgangsmaterial dienende 1,7-Dihydroxynaphthalin-4-sulfonsäure ist jedoch schwer zugänglich, so daß die Durchführung des Gesamtverfahrens sehr aufwendig ist. Außerdem ist die Diazotierung der Aminozwischenstufe nur unter definierten $P_H$- und Temperaturbedingungen und in Anwesenheit von Schwermetallsalzen in akzeptabler Qualität und in nur geringer Ausbeute möglich.

Die Ester und Amide der 7-Methoxy-1,2-naphthochinon-(2)-diazid-4-sulfonsäure sind wegen der längerwelligen Verschiebung ihrer Absorption und ihres hohen Umkehrpotentials hervorragend für die Verwendung in Photoresistschichten geeignet, die im g-line (436 nm) - und i-line (365 nm)-Bereich strukturiert und sowohl positiv als auch negativ verarbeitet werden können.

Aufgabe der Erfindung war es deshalb, ein Verfahren zur Herstellung von in 7-Position substituierten 1,2-Naphthochinon-(2)-diazid-4-sulfonsäuren und deren Derivaten anzugeben, welches die Nachteile der bekannten Verfahren vermeidet, und wodurch die herstellbaren Verbindungen wie Säuren, Salze und die für

Photolacke hieraus bevorzugt herstellbaren Ester und Amide nach produktionstechnisch einfachen Verfahrensschritten in guter Ausbeute ohne zusätzliche Reinigung der Zwischenstufen hergestellt werden können.

Die Erfindung betrifft demgemäß ein Verfahren zur Herstellung von substituierten 1,2-Naphthochinon-(2)-diazid-4-sulfonsäuren und ihren Salzen der allgemeinen Formel A

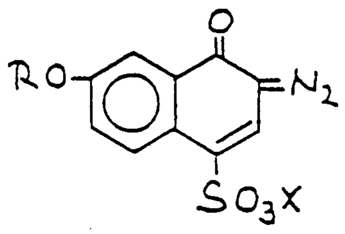

A

in der

R = eine Alkyl-, Epoxyalkyl-, Alkylcarbonyl- oder Alkylsulfonylgruppe, deren Kohlenstoffketten durch Sauerstoffatome unterbrochen sein können,
- eine gegebenenfalls substituierte Aralkyl-, Arylcarbonyl- oder Arylsulfonylgruppe und

X = Wasserstoff, Metall oder eine Ammoniumgruppe

bedeuten, bei dem man

1) 2,7-Dihydroxynaphthalin nitrosiert,

2) das entstandene 2,7-Dihydroxy-1-nitrosonaphthalin (I) mit Alkalihydrogensulfit sulfoniert und die gebildete Bisulfit-Additionsverbindung ohne Zwischenisolierung in saurer Lösung zur 2,7-Dihydroxy-1-aminonaphthalin-4-sulfonsäure (II) reduziert,

3) diese zur 7-Hydroxy-1,2-naphthochinon-4-sulfonsäure (III) oxidiert und als Salz abscheidet,

4) das Salz mit einem Arylsulfonsäurehydrazid zu dem entsprechenden Salz der 7-Hydroxy-1,2-naphthochinon-(2)-diazid-4-sulfonsäure (IV) umsetzt und

5) das Salz, vorzugsweise das Alkalisalz, in wäßrigem Alkali bei Temperaturen zwischen 20 und 30 °C und einem pH-Wert von 11 -12 mit einem Alkylierungs- oder Acylierungsmittel zu dem entsprechenden Salz der in 7-Position substituierten Verbindung der allgemeinen Formel A umsetzt und dieses isoliert.

Vorzugsweise betrifft das Verfahren die Herstellung von Verbindungen der Formel A in der

R eine Alkylgruppe mit 1 bis 6 Kohlenstoffatomen, bevorzugt mit 1 bis 4, insbesondere mit 1 oder 2 Kohlenstoffatomen, eine Epoxyalkylgruppe mit 3 bis 6 Kohlenstoffatomen, eine Alkylcarbonylgruppe mit 2 bis 6 Kohlenstoffatomen oder eine Alkylsulfonylgruppe mit 1 bis 4 Kohlenstoffatomen, deren Kohlenstoffketten durch wenigstens ein Sauerstoffatom unter brochen sein können,
eine Aralkylgruppe mit 7 bis 10 Kohlenstoffatomen, eine Arylcarbonylgruppe mit 7 bis 10 Kohlenstoffatomen, eine Arylsulfonylgruppe mit 6 bis 10 Kohlenstoffatomen, die durch Alkyl-, Trihalogenalkyl-, Alkoxy-, Alkylcarbonyl-, Alkylsulfonylgruppen oder Halogen substituiert sein können, und

X Alkali- oder Erdalkalimetall, z.B. Kalium oder Natrium oder die Ammoniumgruppe bedeuten.

Das erfindungsgemäße Verfahren verläuft nach folgendem Reaktionsschema.

Reaktionsschema

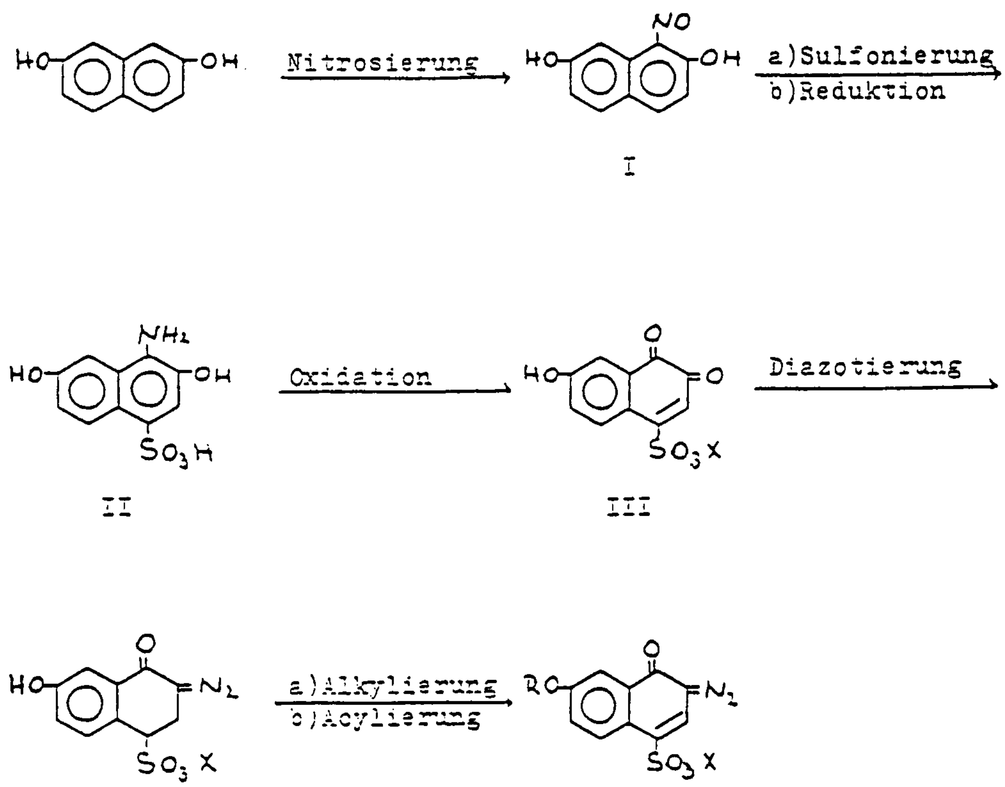


I

II

III

IV

V = A

Ein besonderer Vorteil des erfindungsgemäßen Verfahrens ist, daß zusätzliche Reinigungsschritte der einzelnen Zwischenstufen entfallen und daß damit eine gute Ausbeute am Endprodukt gemäß der allgemeinen Formel A gewährleistet ist.

Die Verbindungen der allgemeinen Formel A können einerseits als lichtempfindliche Verbindungen in strahlungsfindlichen Gemischen eingesetzt werden und andererseits können sie über die Zwischenstufe ihrer Sulfonsäurechloride in bekannter Weise durch Kondensation mit aromatischen Mono- oder Polyhydroxyverbindungen oder mit aromatischen, vorzugsweise mehrkernigen primären oder sekundären Aminen zu den entsprechenden Estern bzw. Amiden umgesetzt und in lichtempfindlichen Gemischen und Materialien eingesetzt werden.

Das erfindungsgemäße Herstellungsverfahren geht aus von handelsüblichem 2,7-Dihydroxynaphthalin, das in bekannter Weise durch Nitrosierung mit Natriumnitrit bei Temperaturen zwischen 0 und 5 °C entweder in mineralsaurer wäßriger Suspension (Clausius, B. 23, 517 (1890); Leonhardt & Co DE-PS 55 204 (1889)) oder in essigsaurer Lösung (Kaufler u. Bräuer, B. 40, 3275 (1907) zu 2,7-Dihydroxy-1-nitrosonaphthalin (I), bzw. dem tautomeren 7-Hydroxy-1,2-naphthochinon-(1)-oxim (I a), umgesetzt werden kann. Nach beiden bekannten Verfahren sind Ausbeute und Qualität des 2,7-Dihydroxy-1-nitrosonaphthalins (I) nicht zufriedenstellend.

Völlig überraschend und in nahezu quantitativer Umsetzung und sehr guter Qualität verläuft jedoch die Nitrosierung von 2,7-Dihydroxynaphthalin analog der von Gates und Webb in J. Am. Chem. Soc. 80, 1186 (1958) für die Herstellung von 6-Methoxy-1-nitroso-2-naphthol beschriebenen Methode. Hiernach wird eine feindisperse Suspension von 2,7-Dihydroxynaphthalin, die man durch Ausfällen von 2,7-Dihydroxynaphthalin mit Eis aus einer warmen, essigsauren Lösung erhält, unter kräftigem Rühren bei + 5 bis - 10 °C mit Natriumnitrit versetzt. Die entstandene dunkelrote mikrokristalline Nitroso-/Oximverbindung (I/Ia) wird abgesaugt, mit Wasser neutral gewaschen und gegebenenfalls getrocknet. Das nach dieser Verfahrensweise erhaltene Reaktionsprodukt wird ohne Reinigung, vorzugsweise als wasserfeuchtes Produkt weiterverarbeitet.

Die Nitrosierung wird erfindungsgemäß in essigsaurer, wäßriger Suspension mit Alkalinitrit bei Temperaturen zwischen + 5 und - 10 °C durchgeführt und die Nitrosoverbindung isoliert.

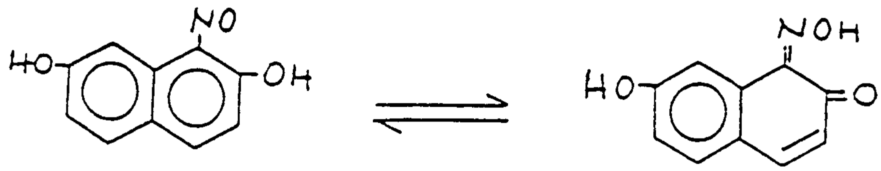

I

I a

In der zweiten Reaktionsstufe wird das in der Regel noch wasserfeuchte 2,7-Dihydroxy-1-nitrosonaphthalin (I), das in der tautomeren Oximform (Ia) reagiert, nach Böninger, Ber. 27,3050 (1894) zunächst in handelsüblicher 37 %iger wäßriger Natriumhydrogensulfitlösung suspendiert und bei 20 - 25 °C bis zur vollständigen Lösung gerührt. Die hierbei entstandene Bisulfit-Additionsverbindung (IIa) wird nicht isoliert, sondern die braune Reaktionslösung mit Salzsäure angesäuert und auf ca. 25 - 60 °C erwärmt, wobei durch Reduktion der Nitrosogruppe und Aromatisierung des cycloaliphatischen Ringsystems die 2,7-Dihydroxy-1-aminonaphthalin-4-sulfonsäure (II) gebildet wird. Man isoliert die Aminonaphtholsulfonsäure (II), indem man die entstandenen hellgrauen Kristalle absaugt, diese auf der Filternutsche zuerst mit Wasser und dann mit Methanol wäscht und gegebenenfalls trocknet. Das so hergestellte Reaktionsprodukt wird ohne weitere Reinigung, gegebenenfalls noch feucht, für die nächste Reaktionsstufe eingesetzt. Die Ausbeute an Reaktionsprodukt II liegt bei 90 - 95 % d. Theorie.

Die Sulfonierung wird in der Regel mit Alkalihydrogensulfit in wäßriger Phase in einem pH-Bereich von 5 - 7 durchgeführt und die entstandene Bisulfit-Additionsverbindung ohne Zwischenisolierung in mineralsaurer wäßriger Lösung bei Temperaturen zwischen 20 und 60 °C zu der entsprechenden Aminosulfonsäure reduziert.

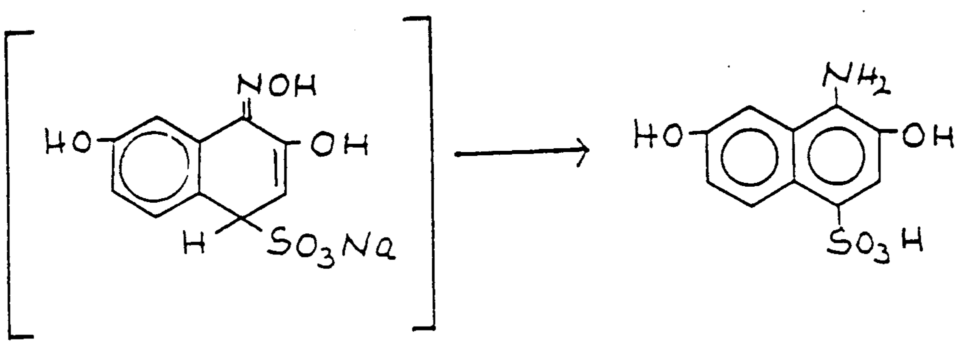

II a

II

Vorteilhafter bei der praktischen Durchführung der Reaktion ist die Verwendung von festem, stabilerem Natriumdisulfit anstelle der käuflichen Natriumhydrogensulfit-Lösung.

In der dritten Reaktionsstufe wird die 2,7-Dihydroxy-1-aminonaphthalin-4-sulfonsäure (II) mit einem Oxidationsmittel zu 7-Hydroxy-1,2-naphthochinon-4-sulfonsäure (III) oxidiert.

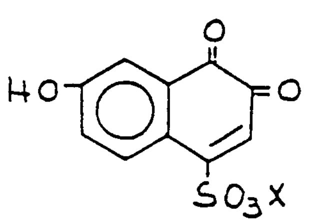

III

Geeignete Oxidationsmittel sind z.B. Kaliumperoxidisulfat ($K_2S_2O_8$), Cr(VI)-oxid, Pb(IV)-oxid, Fe(III)-chlorid, Chlor, Brom oder salpetrige Säure. Vorzugsweise wird jedoch verdünnte Salpetersäure als Oxidationmittel verwendet. Die Isolierung der 7-Hydroxy-1,2-naphthochinon-4-sulfonsäure (III) erfolgt üblicherweise als Ammonium-oder als Alkalisalz.

Bei Verwendung von Salpetersäure verfährt man zweckmäßigerweise analog der von Martin und Fieser in Org. Synth. Coll. Vol. III, 633 (1955) beschriebenen Verfahrensweise. Die Oxidation erfolgt vorzugsweise bei Temperaturen zwischen 15 und 25 °C, wobei man in der Regel die feste Aminonaphtholsulfonsäure unter Rühren portionsweise in die Salpetersäure einträgt. Man arbeitet zweckmäßigerweise mit verdünnter Salpetersäure, um das als Suspension vorliegende Reaktionsgemisch rührfähig zu halten und die exotherm ablaufende Oxidation durch Außenkühlung besser auf Raumtemperatur begrenzen zu können. Besonders bewährt hat sich eine 15 bis 25, vorzugsweise 18 - 22 %ige wäßrige Salpetersäure als Oxidationsmittel. Die entstandene 7-Hydroxy-1,2-naphthochinon-4-sulfonsäure (III) wird vorzugsweise als Ammonium- oder Kaliumsalz durch Aussalzen mit Ammonium- bzw. Kaliumchlorid gefällt und durch Filtration isoliert. Der Filterrückstand wird mit einer gesättigten Ammonium- bzw. Kaliumchloridlösung und anschließend mit Ethanol gewaschen und gegebenenfalls bei 20 - 40 °C getrocknet. Die auf diese Weise hergestellten Salze der 7-Hydroxy-1,2-naphthochinon-4-sulfonsäure enthalten in der Regel geringe Anteile (ca. 2 - 3 %) an 7-Hydroxy-1,2-naphthochinon-(1)-diazid-4-sulfonsäure (IV a). Diese entsteht in einer Nebenreaktion durch Diazotierung der eingesetzten 2,7-Dihydroxy-1-aminonaphthalin-4-sulfonsäure mit salpetriger Säure, die bei Anwendung von Salpetersäure im Oxidationsmedium vorhanden ist. Dieses nur in geringer Menge gebildete Nebenprodukt stört jedoch die nachfolgende Umsetzung zur 7-Hydroxy-1,2-naphthochinon-(2)-diazid-4-sulfonsäure (IV) nicht, da die Salze der isomeren 7-Hydroxy-1,2-naphthochinon-(1)-diazid-4-sulfonsäure (IV a) besser löslich sind als die gewünschten Salze der 7-Hydroxy-1,2-naphthochinon-(2)-diazid-4-sulfonsäure (IV). Bei Anwendung von höher konzentrierter Salpetersäure steigt der Anteil an 7-Hydroxy-1,2-naphthochinon-(1)diazid-4-sulfonsäure (IV a).

Dementsprechend führt man vorzugsweise die Oxidation mit wäßriger Salpetersäure von 15 bis 25 Gewichtsprozent bei Temperaturen zwischen 15 und 25 °C durch, scheidet die entstandene Naphthochinonsulfonsäure als Ammonium- oder Kaliumsalz ab und isoliert sie.

Deutlich größere Mengen (ca. 20 - 40 %) an 7-Hydroxy-1,2-naphthochinon-(1)-diazid-4-sulfonsäure (IV a) entstehen nach der von Böninger in Ber. 27, 3050, (1894) beschriebenen Oxiationsmethode in verdünnter Salzsäure mit Natriumnitrit bei 0 bis 5 °C. Aus der rotbraunen Lösung werden nach dieser bekannten Verfahrensweise durch Aussalzen mit Kaliumchlorid die Kaliumsalze der gewünschten 7-Hydroxy-1,2-naphthochinon-4-sulfonsäure (III) und der unerwünschten 7-Hydroxy-1,2-naphthochinon-(1)-diazid-4-sulfonsäure (IV a) zusammen ausgefällt. Eine Trennung der Kaliumsalze dieser beiden Sulfonsäuren durch fraktionierte Kristallisation ist grundsätzlich möglich, jedoch sehr verlustreich. Für ein technisches Produktionsverfahren ist diese Oxidationsmethode deshalb ungeeignet.

In der vierten Reaktionsstufe werden das Ammonium- oder die Alkalisalze, vorzugsweise das Kaliumsalz der 7-Hydroxy-1,2-naphthochinon-4-sulfonsäure (III), mit einem Arylsulfonsäurehydrazid, z.B. p-Toluolsulfonsäurehydrazid, in wäßriger, vorzugsweise jedoch in alkoholischer Suspension z.B., in Methanol, bei Temperaturen zwischen 20 und 70, vorzugsweise 20 und 30 °C, mit hoher Regioselektivität zu den entsprechenden Salzen der 7-Hydroxy-1,2-naphthochinon-(2)-diazid-4-sulfonsäure (IV) umgesetzt. Die noch zu erwartenden isomeren Salze der 7-Hydroxy-1,2-naphthochinon-(1)-diazid-4-sulfonsäure (IV a) entstehen unter diesen Reaktionsbedingungen nur in äußerst geringen Anteilen (ca. 1 - 2 %).

Vorzugsweise wird das Ammonium- oder Kaliumsalz der Naphthochinonsulfonsäure mit p-Toluolsulfonsäurehydrazid in wäßriger oder organischer Phase bei Temperaturen zwischen 20 und 70 °C zu den entsprechenden Salzen der 7-Hydroxy-1,2-naphthochinon-(2)-diazid-4-sulfonsäure umgesetzt und dieses isoliert.

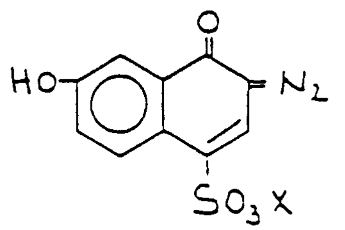

IV

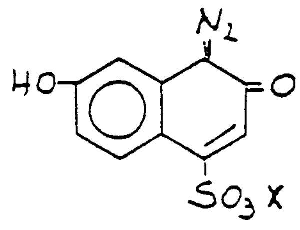

IV a

Die ausgezeichnete Regioselektivität bei dieser Umsetzung ist insofern überraschend, da sich das Isomerenverhältnis der von Horner und Dürckheimer hergestellten und in Chem. Ber. 95, 1206 (1962) beschriebenen substituierten o-Benzochinondiazide von dem Isomerengemisch der o.g. substituierten Naphthochinondiazide z.T. sehr deutlich unterscheidet.

Die 7-Hydroxy-1,2-naphthochinon-(2)-diazid-4-sulfonsäure (IV) und ihre Salze sind in der Literatur bisher noch nicht beschrieben. Durch Reaktionen an der phenolischen Hydroxylgruppe, z.B. Alkylierung oder Acylierung, oder an der Sulfonsäuregruppe, z.B. Chlorierung, gelingt es auf sehr einfache Weise, die substituierten 1,2-Naphthochinon-(2)-diazid-4-sulfonsäuren der allgemeinen Formel A bzw. das noch nicht bekannte 7-Hydroxy-1,2-naphthochinon(2)-diazid-4-sulfonsäurechlorid herzustellen.

Der Austausch eines Sauerstoffatoms bei α-Diketonen durch die Diazogruppe mit Arylsulfonsäurehydrazid ist aus der Literatur als "Bamford-Stevens-Reaktion" bekannt. Bei dieser Umsetzung entsteht intermediär ein Diketonarylhydrazon, das mit oder ohne Verwendung von Alkali unter sehr einfachen und milden Reaktionsbedingungen zu einer α-Diazocarbonylverbindung gespalten werden kann.

In der DE-B 11 26 541 wird diese Reaktion für die Herstellung von 6-Nitro-1,2-naphthochinon-(2)-diazid-4,8-disulfonsäure aus 6-Nitro-1,2-naphthochinon-4,8-disulfonsäure durch Umsetzung mit Arylsulfonsäurehydrazid und anschließende Spaltung des primär gebildeten Hydrazons in wäßrig alkalischem Medium beschrieben. Eine Isolierung des Naphthochinondiazid-Derivats in Substanz ist für das hier beschriebene Herstellungsverfahren für spezielle Azofarbstoffe nicht erforderlich.

Eine weitere Anwendung der "Bamford-Stevens-Reaktion" wird von Süs, Steppan und Dietrich in Liebigs Ann. Chem. 617, 20 (1958) für die Herstellung polycyclischer aromatischer o-Chinondiazide, z.B. Phenanthrenchinon-(9,10)- und Chrysenchinon-(5,6)-diazid, beschrieben. Man erhält diese o-Chinondiazide durch Umsetzung der entsprechenden polycyclischen o-Chinone mit p-Toluolsulfonsäurehydrazid in Ethanol bei Temperaturen zwischen 45 und 60 °C und anschließende Spaltung der intermediär gebildeten Toluolsulfonsäurehydrazone ohne Anwendung von Alkali.

Über die Anwendungsbreite und über die verschiedenen Verfahrensvarianten der "Bamford-Stevens-Reaktion" wird in der Fachliteratur zusammenfassend berichtet (M. Regitz, "Diazoalkane", Kap. 5.3, 129 (1977) oder Houben-Weyl, "Aromatische Diazoniumsalze", Bd. 10/3, 84).

Bei der Herstellung des Ammonium- bzw. Kaliumsalzes der 7-Hydroxy-1,2-naphthochinon-(2)-diazid-4-sulfonsäure (IV) geht man zweckmäßigerweise so vor, daß man die entsprechenden Salze der 7-Hydroxy-1,2-naphthochinon-4-sulfonsäure (III) in Wasser oder einem polaren organischen Lösemittel, vorzugsweise Methanol, suspendiert und bei Temperaturen zwischen 15 und 30 °C das p-Toluolsulfonsäurehydrazid unter Rühren zudosiert. Das Reaktionsgemisch wird unter fortgesetztem Rühren auf 20 und 40 °C erwärmt. Wird ein polares organisches Lösemittel, z.B. Methanol, als Reaktionsmedium verwendet, wandelt sich die anfangs dunkelrote Suspension der Salze der 7-Hydroxy-1,2-naphthochinon-4-sulfonsäure in der Regel ohne erkennbare Lösung in eine gelbe Suspension um. Die nach dieser Verfahrensweise in hohen Ausbeuten anfallenden Ammonium- bzw. Kaliumsalze der 7-Hydroxy-1,2-naphthochinon-(2)-diazid-4-sulfonsäure werden aus dem Reaktionsgemisch durch einfache Filtration isoliert und mit reichlich Methanol gewaschen. Eine zusätzliche Reinigung, z.B. Umfällen oder Umkristallisieren, ist nicht erforderlich.

Die Verwendung eines polaren Lösemittels als Reaktionsmedium, wie z.B. Methanol, ist deshalb besonders vorteilhaft, weil das gebildete Ammonium- oder Kaliumsalz der 7-Hydroxy-1,2-naphthochinon-(2)-diazid-4-sulfonsäure in diesem Lösemittel nur sehr schwer löslich ist. Die geringen Anteile an Verunreinigungen, die vom Einsatzmaterial herrühren, und die bei der Hydrazonspaltung entstehende p-Toluolsulfinsäure sind jedoch in Methanol sehr gut löslich, so daß die Ausbeute an reinem Reaktionsprodukt nach dieser Verfahrensweise sehr hoch ist.

Bei der wäßrigen Verfahrensweise wird die nach der Umsetzung mit p-Toluolsulfonsäurehydrazid entstandene gelbe Suspension durch Erwärmen auf Temperaturen zwischen 40 und 50 °C gelöst, und die gelbbraune Lösung mit einem Absorptionsmittel, z.B. Aktivkohle, von wenig dunklen Produkten befreit und aus dem klaren Filtrat durch Aussalzen mit Ammonium- oder Kaliumchlorid und Abkühlen auf 0 - 5 °C das Reaktionsprodukt ausgefällt. Die Ausbeute und Reinheit ist nach dieser Verfahrensweise geringer als nach der bevorzugten Methode in Methanol als Reaktionsmedium.

Die Umsetzung zu den erfindungsgemäßen substituierten 1,2-Naphthochinon-(2)-diazid-4-sulfonsäuren der allgemeinen Formel A,

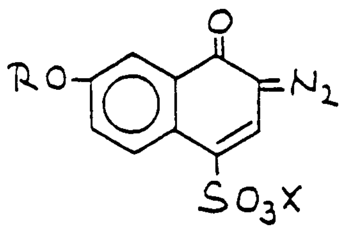

A

in der

R und X die im Anspruch 1 angegebene Bedeutung haben, gelingt völlig überraschend in sehr hoher Ausbeute und ausgezeichneter Reinheit aus den Salzen, vorzugsweise dem Kaliumsalz, der 7-Hydroxy-1,2-naphthochinon-(2)-diazid-4-sulfonsäure (IV), indem man dieses in wäßrig alkalischer Lösung bei Temperaturen zwischen 20 und 50 °C, vorzugsweise zwischen 20 und 25 °C, bei einem pH-Wert von 11 - 12 mit einem Alkylierungsmittel, z.B. einem Alkylhalogenid, Aralkylhalogenid, Epoxialkylhalogenid oder Sulfonsäureester oder mit einem Acylierungsmittel, z.B. einem aromatischen Carbonsäure- oder Sulfonsäurehalogenid umsetzt und die in der Regel kristallin anfallenden Reaktionsprodukte durch Filtration isoliert. Eine zusätzliche Reinigung der Rohprodukte ist für eine Weiterverarbeitung in den meisten Fällen nicht erforderlich.

Als Alkylierungsmittel für die Herstellung der 7-Alkoxy-1,2-naphthochinon-(2)-diazid-4-sulfonsäuren der allgemeinen Formel A verwendet man vorzugsweise aliphatische Sulfonsäureester, etwa Dialkylsulfat wie Dimethyl- oder Diethylsulfat. Grundsätzlich sind jedoch auch aromatische Sulfonsäureester, z.B. p-Toluolsulfonsäureester geeignet, besonders in Anwesenheit von "Phasentransfer-Katalysatoren", z.B. Tetrabutylammoniumbromid oder Benzyltriethylammoniumbromid.

Für die Herstellung der 7-Epoxyalkoxy-1,2-naphthochinon-(2)-diazid-4-sulfonsäuren der allgemeinen Formel A verwendet man als Alkylierungsmittel Epoxyalkylhalogenide, vorzugsweise Epoxypropylbromid oder Epichlorhydrin, für die Herstellung der 7-Arylalkyl-1,2-naphthochinon-(2)-diazid-4-sulfonsäuren der allgemeinen Formel A Aralkylhalogenide, z.B. Benzylbromid oder Benzylchlorid.

Als Acylierungsmittel für die bevorzugten 7-Arylcarbonyloxy- und 7-Arylsulfonyloxyl-1,2-naphthochinon-(2)-diazid-4-sulfonsäuren der allgemeinen Formel A werden Arylcarbonsäurehalogenide, z.B. Benzoylchlorid, 2-Methyl- oder 4-Methylbenzoylchlorid oder Arylsulfonsäurehalogenide, z.B. Benzol- oder p-Toluolsulfonsäurechlorid, eingesetzt.

Die alphatischen 7-Alkylcarbonyloxy-1,2-naphthochinon-(2)-diazid-4-sulfonsäuren der allgemeinen Formel A erhält man besser durch Umsetzung der 7-Hydroxy-1,2-naphthochinon-(2)-diazid-4-sulfonsäure mit Alkylcarbonsäurechloriden oder Alkylcarbonsäureanhydriden in Eisessig in Anwesenheit von wasserfreiem Zinkchlorid.

Für den überraschend glatten Verlauf der Alkylierungs-und Acylierungsreaktion ist die hohe Stabilität der 7-Hydroxy-1,2-naphthochinon(2)-diazid-4-sulfonsäure in wäßrig alkalischer Lösung verantwortlich. Überraschend ist außerdem, daß 7-Hydroxy-1,2-naphthochinon-(2)-diazid-4-sulfonsäure als substituiertes 2-Naphthol-Derivat in alkalischer Lösung nicht zu einem Azofarbstoff kuppelt ("Selbstkupplung").

Die substituierten 1,2-Naphthochinon-(2)-diazidsulfonsäuren der allgemeinen Formel A, vorzusweise die 7-Methoxy-1,2-naphthochinon-(2)-diazid-4-sulfonsäure, sind wertvolle Zwischenprodukte für die Herstellung strahlungsempfindlicher Ester oder Amide. Man erhält diese Derivate aus der 7-Methoxy-1,2-naphthochinon-(2)-diazid-4-sulfonsäure in bekannter Weise durch Chlorierung mit Chlorsulfonsäure oder einem Gemisch aus Chlorsulfonsäure und Thionylchlorid und anschließender Kondensation des entstandenen Sulfonsäurechlorids mit vorzugsweise aromatischem Mono- oder Polyhydroxyverbindungen oder mehrkernigen primären oder sekundären Aminen.

Die strahlungsempfindlichen Ester und Amide der 7-Methoxy-1,2-naphthochinon-(2)-diazid-4-sulfonsäure können vorteilhaft für strahlungsempfindliche Gemische technisch eingesetzt werden, z.B. als Photoresist für die Herstellung von Halbleiterbauelementen in der Mikroelektronik oder für die photomechanische Herstellung von Druckformen oder Farbprüffolien.

Die Erfindung wird durch die folgenden Beispiele näher erläutert, ohne sie hierauf zu beschränken.

Experimenteller Teil

2,7-Dihydroxy-1-nitrosonaphthalin (I)

250 g (1,56 Mol) 2,7-Dihydroxynaphthalin (BAYER) wurden in 875 ml Eisessig bei 0 - 90 °C gelöst und durch Zugabe von 2,5 kg gestoßenem Eis wieder ausgefällt, wobei eine sehr feinteilige, hellbeige, viskose Suspension entstand und die Temperatur auf -10 bis -12 °C absank. Unter gutem Rühren und Außenkühlung wurden in diese Suspension 107,88 g (1,56 Mol) festes Natriumnitrit portionsweise eingetragen, eine Stunde nachgerührt und dann weitere 10,7 g (0,56 Mol) festes Natriumnitrit zudosiert. Anschließend wurde noch zwei Stunden bei - 8 bis - 5 °C nachgerührt, das in dunkelroten Kristallen angefallene 2,7-Dihydroxy-1-nitrosonaphthalin abgesaugt und auf der Nutsche gut abgepreßt. Das noch nutschenfeuchte Reaktionsprodukt wurde dann in 5 l Wasser von 20 - 25 °C ca. eine Stunde gerührt, erneut abgesaugt, gut abgepreßt und 24 Stunden im Umlufttrockenschrank bei 20 - 25 °C getrocknet.

Ausbeute: 294 g Reinprodukt (= 99,6 % d. Theorie)

Kenndaten:

Aspekt: dunkelrotes feinteiliges Pulver
MP: 140 - 145 °C (Aufhellung)
≧ 195 °C (Verkohlung ohne zu schmelzen)

Elementaranalyse

Bruttoformel: $C_{10}H_7O_3N$
MG: 189

| | C | H | N |
|---|---|---|---|
| Ber.: | 63,5 | 3,7 | 7,4 |
| Gef.: | 63,7 | 3,7 | 7,1 |

2,7-Dihydroxy-1-aminonaphthalin-4-sulfonsäure x $2H_2O$ (II)

200 g (1,06 Mol) 2,7-Dihydroxy-1-nitrosonaphthalin (I) wurden in 1,75 l Wasser von 20 - 25 °C suspendiert und unter Rühren 67 g (0.8 Mol) Natriumhydrogencarbonat portionsweise langsam zugegeben, wobei anfängliches Schäumen durch Zugabe von 1 ml n-Octanol verhindert wurde. Anschließend wurden 225 g (1,2 Mol) Natriumdisulfit ($Na_2S_2O_5$) zugesetzt und 17 Stunden bei 20 - 25 °C gerührt, wobei sich die anfangs rote Suspension in eine braune Lösung umwandelte. Anschließend wurde durch Filtration der Lösung über Aktivkohle von wenig dunklen schmierigen Produkten abgetrennt, das jetzt klare braune Filtrat mit 660 ml 37 %iger Salzsäure bis zur kongosauren Reaktion versetzt und unter Rühren 75 Minuten auf 40 - 50 °C erwärmt. Unter $SO_2$-Entwicklung fiel bereits während dieser Zeit ein Teil des Reaktionsproduktes aus. Nach Abkühlen des Reaktionsgemisches auf 20 - 25 °C und 24-stündigem Stehen wurden die ausgefallenen hellgrauen Kristalle abgesaugt, gut abgepreßt und auf der Filternutsche zuerst mit 500 ml Wasser und dann mit 1 l Methanol gewaschen bis die ablaufende Methanolphase nur noch schwach gelb gefärbt war. Nach der Trocknung im Umlufttrockenschrank bei 20 - 25 °C wurde die 2,7-Dihydroxy-1-aminonaphthalin-4-sulfonsäure, die noch zwei Mol Kristallwasser enthielt, als hellgraues Pulver erhalten.

Ausbeute: 288 g Reinprodukt (= 90,5 % d. Theorie)

Kenndaten:

Aspekt: hellgraues Pulver
MP: ≧ 275 °C (Z)

Elementaranalyse

Bruttoformel: $C_{10}H_9O_5NS \times 2H_2O$
MG: 291

| | C | H | N | S | $H_2O$ |
|---|---|---|---|---|---|
| Ber.: | 41,2 | 4,5 | 4,8 | 11,0 | 12,4 |
| Gef.: | 41,8 | 4,2 | 4,6 | 10,9 | 12,1 |

7-Hydroxy-1,2-naphthochinon-4-sulfonsäure (K-Salz) x 1 $H_2O$ (III)

219 g (0,662 Mol) 19 %ige Salpetersäure wurden auf 15 °C abgekühlt und 1,5 g (5,2 x $10^{-3}$ Mol) 2,7-Dihydroxy-1-aminonaphthalin- 4-sulfonsäure x $2H_2O$ (II) unter Rühren eingetragen. Nach Starten der Oxidationsreaktion mit ca. 1 ml 65 % iger Salpetersäure wurden unter fortgesetztem Rühren jetzt in die entstandene dunkelrote Lösung die restlichen 148,5 g (0,51 Mol) 2,7-Dihydroxy-1-aminonaphthalin-4-sulfonsäure x $2H_2O$ bei 15 - 20 °C innerhalb von zwei Stunden portionsweise langsam eingetragen. Anfängliches Schäumen konnte durch Zugabe einiger Tropfen n-Octanol stark reduziert werden. War die gesamte 2,7-Dihydroxy-1-aminonaphthalin-4-sulfonsäure x $2H_2O$ eindosiert, wurde noch 30 Minuten bei 15 - 20 °C nachgerührt und dann die dunkelrote Suspension in 1,6 l Wasser von 50 °C eingerührt. Es entstand eine klare dunkelrote Lösung. In diese Lösung wurden 170 g Kaliumchlorid in Anteilen von je ca. 25 g eingetragen. Bereits bei Zugabe der ersten 25 g Kaliumchlorid begann die Abscheidung roter glänzender Kristalle aus der Lösung. Das Reaktionsgemisch wurde auf 0 bis 5 °C abgekühlt, nach zwei Stunden abgesaugt und der Nutscheninhalt zuerst mit 80 ml gesättigte Kaliumchloridlösung, dann mit 160 ml Ethanol gewaschen und gut abgepreßt. Anschließend wurde der Filterrückstand im Umlufttrockenschrank bei 20 - 25 °C getrocknet.

Das so hergestellte rohe 7-Hydroxy-1,2-naphthochinon-4-sulfonsäure-(K-Salz) enthielt neben Kaliumchlorid noch geringe Anteile an 7-Hydroxy-1,2-naphthochinon-(1)-diazid-4-sulfonsäure-(K-Salz) und 1 Mol Kristallwasser.

Das reine 7-Hydroxy-1,2-naphthochinon-4-sulfonsäure-(K-Salz) erhielt man durch Umlösen des Rohprodukts aus Wasser.

Ausbeute: 148 g Rohprodukt (89 %ige Qualität)
= 131,7 g Reinprodukt (100 %ige Qualität),
d.h. 82,4 % d. Theorie

Kenndaten:

Aspekt: dunkelrotes kristallines Pulver
MP: ≧ 275 °C (Z)

Elementaranalyse

Bruttoformel: $C_{10}H_5O_6SK \times 1H_2O$
MG: 310

| | C | H | S | $H_2O$ |
|---|---|---|---|---|
| Ber.: | 38,7 | 2,3 | 10,3 | 5,8 (%) |
| Gef.: | 38,8 | 2,1 | 9,9 | 6,1 (%) |

7-Hydroxy-1,2-naphthochinon-4-sulfonsäure ($NH_4$-Salz) x $1H_2O$ (III a)

Das Ammoniumsalz der 7-Hydroxy-1,2-naphthochinon-4-sulfonsäure wurde dadurch erhalten, daß die nach der Salpetersäure-Oxidation entstandene Suspension ouf 0 - 5 °C abgekühlt und unter Rühren mit gesättigter Ammoniumchloridlösung versetzt wurde. Nach zwei Stunden wurde die rote Suspension abgesaugt, der Filterrückstand auf der Nutsche zuerst mit gesättigter Ammoniumchloridlösung und anschließend mit Ethanol gewaschen, gut abgepreßt und im Umlufttrockenschrank bei 20 - 25 °C getrocknet.

Das so erhaltene 7-Hydroxy-1,2-naphthochinon-4-sulfonsäure ($NH_4$-Salz) enthielt neben Ammoniumchlorid noch geringe Anteile an 7-Hydroxy-1,2-naphthochinon-(1)-diazid-4-sulfonsäure (K-Salz) und 1 Mol Kristallwasser.

Das reine 7-Hydroxy-1,2-naphthochinon-4-sulfonsäure-(K-Salz) erhielt man durch Umlösen des Rohprodukts aus Wasser.

Ausbeute: 125 g Rohprodukt (93,40 %ige Qualität)
= 117,7 g Reinprodukt (100 %ige Qualität),
d.h. 78,5 % d. Theorie

Kenndaten:

Aspekt: dukelrotes kristallines Produkt
MP: ≧ 215 °C (Z)

Elementaranalyse

Bruttoformel: $C_{10}H_9NO_6S$ x $1H_2O$
MG: 289

| | C | H | N | S | $H_2O$ |
|---|---|---|---|---|---|
| Ber.: | 41,5 | 3,8 | 4,8 | 11,1 | 6,2 |
| Gef.: | 38,8 | 2,1 | 5,1 | 10,7 | 5,9 |

7-Hydroxy-1,2-naphthochinon-(2)-diazid-4-sulfonsäure (K-Salz) x 1 $H_2O$ (IV)

131,4g (0,377 Mol) 89 %iges 7-Hydroxy-1,2-naphthochinon-4-sulfonsäure (K-Salz) x 1 $H_2O$ wurden unter Rühren in eine Suspension aus 88,2 g (0,47 Mol) p-Toluolsulfonsäurehydrazid und 900 ml Methanol innerhalb von 25 Minuten portionsweise eingetragen und 3 Stunden bei 20 - 28 °C weitergerührt, wobei sich die dunkelrote Suspension in eine gelbe umwandelte. Diese wurde anschließend auf 0 bis 5 °C abgekühlt, das rohe gelbe Reaktionsprodukt abgesaugt, auf der Filternutsche zweimal mit je 100 ml Ethanol gewaschen und der Filterrückstand 16 Stunden im Umlufttrockenschrank bei 50 - 55 °C getrocknet. Das rohe Reaktionsprodukt enthielt geringe Anteile an 7-Hydroxy-1,2-naphthochinon-(1)-diazid-4-sulfonsäure (K-Salz) und 1 Mol Kristallwasser. Durch Umlösen des Rohprodukts aus Ethanol/Wasser wurde das reine 7-Hydroxy-1,2-naphthochinon-(2)-diazid-4-sulfonsäure (K-Salz) erhalten.

Ausbeute: 112,5 g Rohprodukt (95,2 %ige Qualität)
=107,1 g Reinprodukt (100 %ige Qualität),
d.h. 93,5 % d. Theorie

Kenndaten:

Aspekt: hellgelbes kristallines Pulver
MP: ≧ 165 °C (Z)

Elementaranalyse

Bruttoformel: $C_{10}H_5N_2O_5SK$ x $1H_2O$
MG: 304

| | C | H | N | S | $H_2O$ |
|---|---|---|---|---|---|
| Ber.: | 37,3 | 2,2 | 8,7 | 9,9 | 5,6 |
| Gef.: | 37,0 | 2,0 | 8,5 | 9,6 | 5,3 |

7-Methoxy-1,2-naphthochinon-(2)-diazid-4-sulfonsäure (K-Salz) x 1 $H_2O$ (V/A)

21,28 g (0,067 Mol) 95,2 %iges 7-Hydroxy-1,2-naphthochinon-(2)-diazid-4-sulfonsäure (K-Salz) x 1 $H_2O$ wurden in 140 ml Wasser und 36 ml (0,072 Mol) 2 n Kalilauge gelöst. In die entstandene orangerote Lösung (pH-Wert: 12,08) wurden dann bei 20 - 25 °C unter Rühren 13,23 g (0,105 Mol) Dimethylsulfat gegeben.

Die sofort einsetzende Methylierungsreaktion wurde durch einen Abfall des pH-Wertes der Lösung angezeigt (pH-Meter). Durch Zutropfen von 16,8 ml (0,034 Mol) 2 n Kalilauge über eine Meßpipette wurde der pH-Wert der Lösung in einem Bereich zwischen 11 und 12 gehalten ("pH-kontrollierte Reaktion").

Nach ca. 30 Minuten schieden sich aus der gelbbraunen Lösung hellgelbe perlmuttglänzende Blättchen ab. Die entstandene Suspension wurde zuerst 1,5 Stunden bei 0 bis 2 °C gerührt, wobei sich ein pH-Wert auf 11,5 - 11,7 einstellte. Anschließend wurde abgesaugt, der Filterrückstand zuerst mit 20 ml gesättigter Kaliumchloridlösung und dann mit 15 ml Eiswasser gewaschen und anschließend im Umlufttrockenschrank bei 20 - 25 °C getrocknet. Das rohe Reaktionsprodukt enthielt noch 1 Mol Kristallwasser.

Das reine 7-Methoxy-1,2-naphthochinon-(2)-diazid-4-sulfonsäure (K-Salz) x 1 $H_2O$ kann durch Umlösen des Rohprodukts aus Wasser erhalten werden.

Ausbeute: 19,95 g Rohprodukt (91,4 %ige Qualität)
= 18,23g Reinprodukt (100 %ige Qualität),
d.h. 81 % d. Theorie

Kenndaten:

Aspekt: gelbes kristallines Pulver
MP: ≧ 150 °C (Z)

Elementaranalyse

Bruttoformel: $C_{11}H_7N_2O_5SK$ x $1H_2O$
MG: 336

| | C | H | N | S | $H_2O$ |
|---|---|---|---|---|---|
| Ber.: | 39,3 | 2,7 | 8,3 | 9,5 | 5,4 |
| Gef.: | 38,8 | 2,5 | 8,2 | 9,5 | 5,3 |

Auf analoge Weise wurden durch Umsetzung von 7-Hydroxy-1,2-naphthochinon-(2)-diazid-4-sulfonsäure (K-Salz) x 1 $H_2O$ (IV) mit verschiedenen Veretherungsmitteln (B), z.B. Diethylsulfat, Epibromhydrin, Benzylbromid, oder Veresterungsmitteln (C), z.B. Benzoylchlorid, p-Toluolsulfonsäurechlorid, die entsprechenden in der 7-Position durch Ether- bzw. Esterreste substituierte 1,2-Naphthochinon-(2)-diazid-4-sulfonsäurederivate der allgemeinen Formel A erhalten. Das 7-Acetyloxy-Derivat der allgemeinen Formel A konnte durch Umsetzung von 7-Hydroxy-1,2-naphthochinon-(2)-diazid-4-sulfonsäure (K-Salz) x 1 $H_2O$ mit Essigsäureanhydrid in Eisessig und wasserfreiem Zinkchlorid hergestellt werden.

In der folgenden Tabelle 1 wurden einige der erfindungsgemäßen Verbindungen der allgemeinen Formel A zusammengestellt.

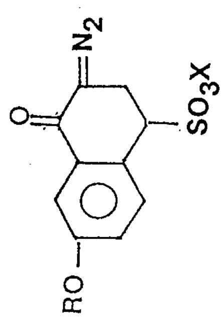

A

| R | X | Veretherungsmittel B<br>Veresterungsmittel C | Molverhältnis IV:B | Molverhältnis IV:C | Reaktionszeit (Std.) | Fp. (°C) | Ausbeute (%) | Elementar-Analyse C | H | N | S | $H_2O$ |
|---|---|---|---|---|---|---|---|---|---|---|---|---|
| $CH_3$- | K | $(CH_3O)_2SO_2$ (B) | 1:1,5 | - | 1,5 | 150(Z.) | 85 | B.39,3<br>G.38,4 | 2,7<br>2,2 | 8,3<br>8,2 | 9,5<br>9,5 | 5,4<br>5,3 |
| $C_2H_5$- | K | $(C_2H_5O)_2SO_2$ (B) | 1:1,1 | - | 6 | 150(Z.) | 70 | B.41,1<br>G.40,6 | 3,1<br>2,6 | 8,0<br>7,7 | 9,1<br>9,1 | 5,1<br>3,2 |
| 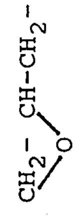 | K | 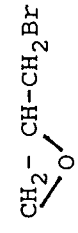 (B) | 1:1,1 | - | 4,5 | 145(Z.) | 55 | B.43,3<br>G.42,8 | 2,5<br>2,5 | 7,8<br>7,8 | 8,9<br>8,7 | 4,8<br>4,4 |
| 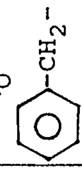 | K | 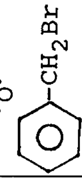 (B) | 1:1,1 | - | 4,5 | 120(Z.) | 62 | B.49,5<br>G.49,6 | 3,2<br>2,9 | 6,8<br>6,6 | 7,8<br>7,6 | 4,4<br>4,2 |
| 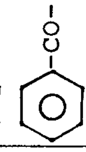 | K | 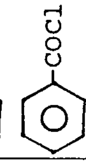 (C) | - | 1:1,15 | 2,5 | 250(Z.) | 90 | B.47,9<br>G.48,0 | 2,6<br>2,3 | 6,6<br>6,5 | 7,5<br>7,3 | 4,4<br>4,0 |
| 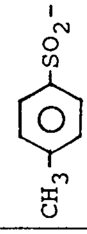 | K | 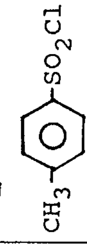 (C) | - | 1:1,15 | 2,5 | 230(Z.) | 83 | B.41,3<br>G.41,3 | 3,0<br>2,7 | 5,7<br>5,5 | 13,0<br>12,2 | 7,9<br>7,4 |
| $CH_3CO$- | K | $(CH_3CO)_2O$ | | | 2 | 155(Z.) | 66 | B.41,6<br>G.41,0 | 2,0<br>2,0 | 8,1<br>7,8 | 9,3<br>9,0 | -<br>- |

7-Methoxy-1,2-naphthochinon-(2)-diazid-4-sulfonsäurechlorid (VI)

In 72,5 ml (1,13 Mol) Chlorsulfonsäure wurden bei 12 - 15 °C innerhalb 35 Minuten 15 g (0,0408 Mol) 91.4 %iges 7-Methoxy-1,2-naphthochinon-(2)-diazid-4-sulfonsäure (K-Salz) x 1 $H_2O$ (V) portionsweise einge-

tragen und anschließend 16,35 ml (0224 Mol) Thionylchlorid zugegeben. Unter $SO_2$- und HCl-Gasentwicklung stieg die Temperatur des Reaktionsgemisches auf 18 - 20 °C an. Es wurde noch 15 Minuten auf 48 - 50 °C erwärmt, das Reaktionsgemisch auf 20 °C abgekühlt und dieses dann unter gutem Rühren in 680 g Eis und 230 ml Wasser vorsichtig eingetropft. Nach 3 - 4 Stunden wurde abgesaugt und der nutschenfeuchte Filterrückstand (ca. 29 g) in 690 ml Aceton gelöst, die gelbbraune Lösung mit 2 g Aktiv-Kohle 5 Minuten bei 22 - 24 °C gerührt, die A-Kohle abfiltriert und das klare Filtrat innerhalb 50 Minuten in ein Gemisch aus 1500 ml Wasser und 50 ml 36 %ige Salzsäure getropft. Nach 2 Minuten wurde abgesaugt und das gut abgepreßte Produkt 16 Stunden über Phosphorpentoxid im Vakuum getrocknet.

Ausbeute: 10,75 g Reinprodukt, d.h. 88,3 % d. Theorie

Kenndaten:

Aspekt: hellgelbes feinteiliges Pulver
MP: ≧ 165 °C (Z), Rotfärbung

Elementaranalyse

Bruttoformel: $C_{11}H_7N_2O_4$ S Cl
MG: 298,5

| | C | H | N | S | Cl |
|---|---|---|---|---|---|
| Ber.: | 44,2 | 2,3 | 9,4 | 10,7 | 11,9 |
| Gef.: | 44,1 | 2,1 | 9,4 | 10,8 | 12,2 |

Kondensationsprodukt aus 7-Methoxy-1,2-naphthochinon-(2)-diazid-4-sulfonsäurechlorid und 2,3,4-Trihydroxybenzophenon (VII)

5,17 g (0,0173 Mol) 7-Methoxy-1,2-naphthochinon-(2)-diazid-4-sulfonsäurechlorid (VI) und 1,265 g (0,0055 Mol) 2,3,4-Trihydroxybenzophenon wurden in 85 ml Acetonitril suspendiert und hierzu unter Rühren innerhalb von 20 Minuten 3,62 g (0,0358 Mol) N-Methylmorpholin gegeben, wobei das Ausgangsmaterial in Lösung ging und die Temperatur von 22 auf 28 °C anstieg. Es wurde noch 1,5 Stunden bei 20 - 22 °C nachgerührt und dann die dunkelbraune Lösung in 500 ml 0,1 n Salzsäure eingetropft. Das ausgefallene Reaktionsprodukt wurde abgesaugt, in 200 ml Aceton gelöst, die Lösung mit Aktiv-Kohle behandelt, die filtrierte klare gelbe Lösung in 500 ml 0,1 n Salzsäure eingetropft, das ausgefallene Produkt abgesaugt, dieses 10 Minuten in 200 ml Ethanol bei 50 - 70 °C digeriert und die Suspension dann auf 20 - 23 °C abgekühlt. Das auf diese Weise gereinigte Reaktionsprodukt wurde abgesaugt und bei 22 - 25 °C im Umlufttrockenschrank getrocknet.

Ausbeute: 5,1 g Kondensationsprodukt

Kenndaten:

Aspekt: hellgelbe, feinpulvrige Substanz
MP: ≧ 150 °C (Z)

HPLC-Analyse

Zusammensetzung des Kondensationsproduktes

4,8 g Trisester des 2,3,4-Trihydroxybenzophenons = 94 % d. Theorie
0,2 g Diester des 2,3,4-Trihydroxybenzophenons = 4 % d. Theorie
0,1 g Monoester des 2,3,4-Trihydroxybenzophenons = 2 % d. Theorie

Elementaranalyse

Bruttoformel: $C_{46}H_{28}N_6O_{16}S_3$ (Triester)
MG: 1016

| | C | H | N | S |
|---|---|---|---|---|
| Ber.: | 54,3 | 2,8 | 8,3 | 9,4 |
| Gef.: | 53,0 | 2,6 | 7,8 | 9,7 |

## Patentansprüche

1. Substituierte 1,2-Naphthochinon-(2)-diazid-4-sulfonsäuren der allgemeinen Formel A

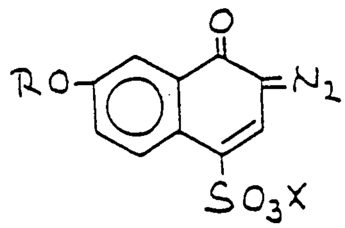

A

in der
R eine Epoxyalkyl-, Alkylcarbonyl- oder Alkylsulfonylgruppe, deren Kohlenstoffketten durch Sauerstoffatome unterbrochen sein können, eine gegebenenfalls substituierte Aralkyl-, Arylcarbonyl- oder Arylsulfonylgruppe
und
X Wasserstoff, Metall oder eine Ammoniumgruppe
bedeuten.

2. Verbindungen nach Anspruch 1, in der
R eine Epoxyalkylgruppe mit 3 bis 6 Kohlenstoffatomen, eine Alkylcarbonylgruppe mit 2 bis 6 Kohlenstoffatomen oder eine Alkylsulfonylgruppe mit 1 bis 4 Kohlenstoffatomen, deren Kohlenstoffketten durch wenigstens ein Sauerstoffatom unterbrochen sein können,
eine Aralkylgruppe mit 7 bis 10 Kohlenstoffatomen, eine Arylcarbonylgruppe mit 7 bis 10 Kohlenstoffatomen, eine Arylsulfonylgruppe mit 6 bis 10 Kohlenstoffatomen, die durch Alkyl-, Trihalogenalkyl-, Alkoxy-, Alkylcarbonyl-, Alkylsulfonylgruppen oder Halogen substituiert sein können, und
X Alkali- oder Erdalkalimetall
bedeuten.

3. Verbindungen nach Anspruch 1 oder 2 worin
X= Kalium oder Natrium
bedeutet.

4. Verfahren zur Herstellung von substituierten 1,2-Naphthochinon-(2)-diazid-4-sulfonsäuren und ihren Salzen der allgemeinen Formel A

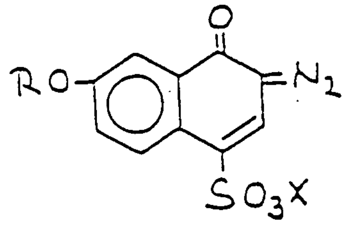

A

in der
R eine Alkyl, Epoxyalkyl-, Alkylcarbonyl- oder Alkylsulfonylgruppe, deren Kohlenstoffketten durch

Sauerstoffatome unterbrochen sein können. eine gegebenenfalls substituierte Aralkyl-, Arylcarbonyl- oder Arylsulfonylgruppe
und
X Wasserstoff, Metall oder eine Ammoniumgruppe bedeuten, bei dem man
1) 2,7-Dihydroxynaphthalin nitrosiert,
2) das entstandene 2,7-Dihydroxy-1-nitrosonaphthalin (I) mit Alkalihydrogensulfit sulfoniert und die gebildete Bisulfit-Additionsverbindung ohne Zwischenisolierung in saurer Lösung zur 2,7-Dihydroxy-1-aminonaphthalin-4-sulfonsäure (II) reduziert,
3) diese zur 7-Hydroxy-1,2-naphthochinon-4-sulfonsäure (III) oxidiert und als Salz abscheidet,
4) das Salz mit einem Arylsulfonsäurehydrazid zu dem entsprechenden Salz der 7-Hydroxy-1,2-Naphthochinon-(2)-diazid-4-sulfonsäure (IV) umsetzt und
5) dieses Salz, vorzugsweise das Alkalisalz, in wäßrigem Alkali bei Temperaturen zwischen 20 und 30 °C und einem pH-Wert von 11 -12 mit einem Alkylierungs- oder Acylierungsmittel zu dem entsprechenden Salz der in 7-Position substituierten Verbindung der allgemeinen Formel A umsetzt und dieses isoliert.

**5.** Verfahren nach Anspruch 4, dadurch gekennzeichnet, daß man die Nitrosierung in essigsaurer wäßriger Suspension mit Alkalinitrit bei Temperaturen zwischen + 5 und - 10 °C durchführt und die Nitrosoverbindung isoliert.

**6.** Verfahren nach Anspruch 4, dadurch gekennzeichnet, daß man die Sulfonierung mit Alkalihydrogensulfit oder Alkalidisulfit in wäßriger Phase in einem pH-Bereich von 5 - 7 durchführt und die entstandene Bisulfit-Additions-Verbindung ohne Zwischenisolierung in mineralsaurer wäßriger Lösung bei Temperaturen zwischen 20 und 60 °C zu der entsprechenden Aminosulfonsäure reduziert.

**7.** Verfahren nach Anspruch 4, dadurch gekennzeichnet, daß man die Oxidation mit wäßriger Salpetersäure von 15 - 25 Gewichtsprozent bei Temperaturen zwischen 15 und 25 °C durchführt und die entstandene Naphthochinonsulfonsäure als Ammonium- oder Kaliumsalz abscheidet und isoliert.

**8.** Verfahren nach Anspruch 4, dadurch gekennzeichnet, daß man das Ammonium- oder Kaliumsalz der Naphthochinonsulfonsäure mit p-Toluolsulfonsäurehydrazid in wäßriger oder organischer Phase bei Temperaturen zwischen 20 und 70 °C zu den entsprechenden Salzen der 7-Hydroxy-1,2-naphthochinon-(2)-diazid-4-sulfonsäure umsetzt und diese isoliert.

**9.** Verfahren nach Anspruch 4, dadurch gekennzeichnet, daß man die Alkylierung mit aliphatischen oder aromatischen Sulfonsäureestern, mit Alkyl-, Aralkyl- oder mit Epoxialkylhalogeniden durchführt.

**10.** Verfahren nach Anspruch 4, dadurch gekennzeichnet, daß man die Acylierung mit einem aromatischen Carbonsäure- oder Sulfonsäurehalogenid durchführt.

**11.** Verfahren nach Anspruch 9, dadurch gekennzeichnet, daß man als Alkylierungsmittel Dialkylsulfat verwendet.

**12.** Verfahren nach Anspruch 9, dadurch gekennzeichnet, daß man als Alkylierungsmittel Epoxypropylbromid oder Benzylbromid verwendet.

**13.** Verfahren nach Anspruch 10, dadurch gekennzeichnet, daß man als Acylierungsmittel Benzoyl- oder p-Toluolsulfonsäurechlorid verwendet.

**14.** Verfahren nach Anspruch 4, in der
R eine Alkylgruppe mit 1 bis 6 Kohlenstoffatomen, eine Epoxyalkylgruppe mit 3 bis 6 Kohlenstoffatomen, eine Alkylcarbonylgruppe mit 2 bis 6 Kohlenstoffatomen oder eine Alkylsulfonylgruppe mit 1 bis 4 Kohlenstoffatomen, deren Kohlenstoffketten durch wenigstens ein Sauerstoffatom unterbrochen sein können, eine Aralkylgruppe mit 7 bis 10 Kohlenstoffatomen, eine Arylcarbonylgruppe mit 7 bis 10 Kohlenstoffatomen, eine Arylsulfonylgruppe mit 6 bis 10 Kohlenstoffatomen, die durch Alkyl-, Trihalogenalkyl-, Alkoxy-, Alkylcarbonyl-, Alkylsulfonylgruppen oder Halogen substituiert sein können, und
X Alkali- oder Erdalkalimetall

bedeuten.

**15.** Verfahren nach Anspruch 14, worin
X Kalium oder Natrium
bedeutet.

**16.** Verwendung der Verbindungen nach der allgemeinen Formel A gemäß Anspruch 1 als Zwischenprodukte für die Herstellung lichtempfindlicher Derivate in lichtempfindlichen Gemischen und Materialien.

## Claims

**1.** A substituted 1,2-naphthoquinone-2-diazide-4-sulfonic acid of the general formula A

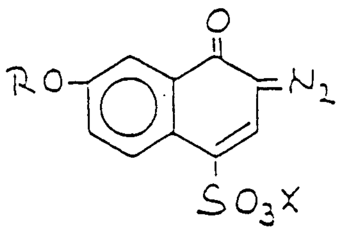

A

in which
- R is an epoxyalkyl, alkylcarbonyl or alkylsulfonyl group, the carbon chains of which may be interrupted by oxygen atoms, or a substituted or unsubstituted aralkyl, arylcarbonyl or arylsulfonyl group and
- X is hydrogen, a metal or an ammonium group.

**2.** A compound as claimed in claim 1, wherein
- R is an epoxyalkyl group having 3 to 6 carbon atoms, an alkylcarbonyl group having 2 to 6 carbon atoms or an alkylsulfonyl group having 1 to 4 carbon atoms, the carbon chains of which groups may be interrupted by at least one oxygen atom, or an aralkyl group having 7 to 10 carbon atoms, an arylcarbonyl group having 7 to 10 carbon atoms or an arylsulfonyl group having 6 to 10 carbon atoms, which groups may be substituted by alkyl, trihalogenoalkyl, alkoxy, alkylcarbonyl or alkylsulfonyl groups or halogen, and
- X is an alkali metal or alkaline earth metal.

**3.** A compound as claimed in claim 1 or 2, wherein
X is potassium or sodium.

**4.** A process for preparing a substituted 1,2-naphthoquinone-2-diazide-4-sulfonic acid and salts thereof of the general formula A

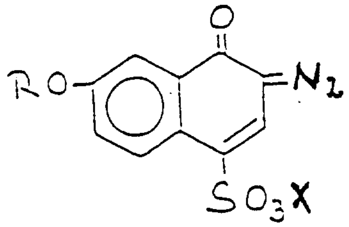

A

in which
- R is an alkyl, epoxyalkyl, alkylcarbonyl or alkylsulfonyl group, the carbon chains of which may be interrupted by oxygen atoms, or a substituted or unsubstituted aralkyl, arylcarbonyl or arylsulfonyl group and
- X is hydrogen, a metal or an ammonium group,

wherein

1) 2,7-dihydroxynaphthalene is nitrosated,

2) the resulting 2,7-dihydroxy-1-nitrosonaphthalene (I) is sulfonated with an alkali metal hydrogen sulfite and the bisulfite addition compound formed is reduced in acidic solution without intermediate isolation to 2,7-dihydroxy-1-aminonaphthalene-4-sulfonic acid (II),

3) the latter is oxidized to 7-hydroxy-1,2-naphthoquinone-4-sulfonic acid (III) and precipitated as a salt,

4) the salt is converted with an arylsulfonic acid hydrazide to the corresponding salt of 7-hydroxy-1,2-naphthoquinone-2-diazide-4-sulfonic acid (IV) and

5) this salt, preferably the alkali metal salt, is converted in aqueous alkali at temperatures between 20 and 30 °C and at a pH of 11 - 12 with an alkylating or acylating agent to the corresponding salt of the compound, substituted in the 7-position, of the formula A, and this salt is isolated.

**5.** The process as claimed in claim 4, wherein the nitrosation is carried out in aqueous suspension containing acetic acid with an alkali metal nitrite at temperatures between +5 and -10 °C and the nitroso compound is isolated.

**6.** The process as claimed in claim 4, wherein the sulfonation is carried out with an alkali metal hydrogen sulfite or alkali metal disulfite in aqueous phase in a pH range of 5 - 7 and the bisulfite addition compound formed is reduced, without intermediate isolation, in an aqueous solution containing a mineral acid at temperatures between 20 and 60 °C to the corresponding aminosulfonic acid.

**7.** The process as claimed in claim 4, wherein the oxidation is carried out with 15 - 25 percent by weight aqueous nitric acid at temperatures between 15 and 25 °C and the naphthoquinonesulfonic acid formed is precipitated and isolated as the ammonium salt or potassium salt.

**8.** The process as claimed in claim 4, wherein the ammonium salt or potassium salt of the naphthoquinonesulfonic acid is converted with p-toluenesulfonic acid hydrazide in aqueous or organic phase at temperatures between 20 and 70 °C to the corresponding salt of 7-hydroxy-1,2-naphthoquinone-2-diazide-4-sulfonicacid and this salt is isolated.

**9.** The process as claimed in claim 4, wherein the alkylation is carried out with aliphatic or aromatic sulfonic acid esters or with alkyl, aralkyl or epoxyalkyl halides.

**10.** The process as claimed in claim 4, wherein the acylation is carried out with an aromatic carboxylic or sulfonic acid halide.

**11.** The process as claimed in claim 9, wherein a dialkyl sulfate is used as the alkylating agent.

**12.** The process as claimed in claim 9, wherein epoxypropyl bromide or benzyl bromide is used as the alkylating agent.

**13.** The process as claimed in claim 10, wherein benzoyl chloride or p-toluenesulfonic acid chloride is used as the acylating agent.

**14.** The process as claimed in claim 4, wherein

R is an alkyl group having 1 to 6 carbon atoms, an epoxyalkyl group having 3 to 6 carbon atoms, an alkylcarbonyl group having 2 to 6 carbon atoms or an alkylsulfonyl group having 1 to 4 carbon atoms, the carbon chains of which groups may be interrupted by at least one oxygen atom, or an aralkyl group having 7 to 10 carbon atoms, an arylcarbonyl group having 7 to 10 carbon atoms or an arylsulfonyl group having 6 to 10 carbon atoms, which groups may be substituted by alkyl, trihalogenoalkyl, alkoxy, alkylcarbonyl or alkylsulfonyl groups or halogen, and

X is an alkali metal or alkaline earth metal.

**15.** The process as claimed in claim 14, wherein

X is potassium or sodium.

16. The use of a compound of the general formula A as claimed in claim 1, as an intermediate for the production of light-sensitive derivatives in light-sensitive mixtures and materials.

**Revendications**

1. Acides 1,2-naphtoquinone-(2)-diazide-4-sulfoniques substitués de formule générale A

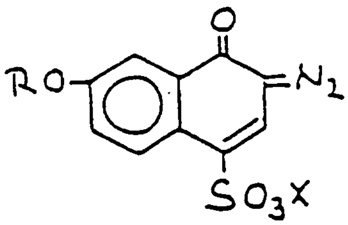

A

dans laquelle
- R représente un groupe époxyalkyle, alkylcarbonyle ou alkylsulfonyle, dont les chaînes carbonées peuvent être interrompues par des atomes d'oxygène, un groupe aralkyle, arylcarbonyle ou arylsulfonyle éventuellement substitué, et
- X représente un atome d'hydrogène, un métal ou le groupe ammonium.

2. Composés selon la revendication 1 dans lesquels
- R représente un groupe époxyalkyle ayant de 3 à 6 atomes de carbone, un groupe alkylcarbonyle ayant de 2 à 6 atomes de carbone ou un groupe alkylsulfonyle ayant de 1 à 4 atomes de carbone, dont les chaînes carbonées peuvent être interrompues par au moins un atome d'oxygène, un groupe aralkyle ayant de 7 à 10 atomes de carbone, un groupe arylcarbonyle ayant de 7 à 10 atomes de carbone, un groupe arylsulfonyle ayant de 6 à 10 atomes de carbone, qui peuvent être substitués par des halogènes ou par des radicaux alkyle, trihalogénoalkyle, alcoxy, alkylcarbonyle ou alkylsulfonyle, et
- X représente un métal alcalin ou alcalino-terreux.

3. Composés selon la revendication 1 ou 2, dans lesquels
- X représente le sodium ou le potassium.

4. Procédé pour la préparation d'acides 1,2-naphtoquinone-(2)-diazide-4-sulfoniques substitués et de leurs sels, de formule générale A

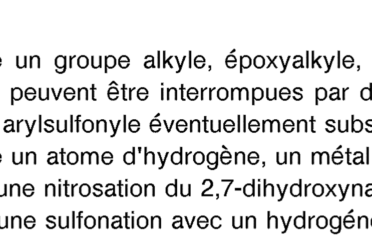

A

dans laquelle
- R représente un groupe alkyle, époxyalkyle, alkylcarbonyle ou alkylsulfonyle, dont les chaînes carbonées peuvent être interrompues par des atomes d'oxygène, un groupe aralkyle, arylcarbonyle ou arylsulfonyle éventuellement substitué, et
- X représente un atome d'hydrogène, un métal ou le groupe ammonium, dans lequel

1) on soumet à une nitrosation du 2,7-dihydroxynaphtalène,

2) on soumet à une sulfonation avec un hydrogénosulfite alcalin le 2,7-dihydroxy-1-nitrosonaphtalène (I) formé et on réduit le composé d'addition bisulfite formé, en solution acide, pour aboutir à l'acide 2,7-dihydroxy-1-amino-naphtalène-4-sulfonique (II),

3) on oxyde ce dernier en l'acide 7-hydroxy-1,2-naphtoquinone-4-sulfonique (III) et on le sépare sous forme de sel, et

4) on fait réagir ce sel avec un arylsulfonhydrazide, pour aboutir au sel correspondant de l'acide 7-hydroxy-1,2-naphtoquinone-(2)-diazide-4-sulfonique (IV), et

5) on fait réagir le sel, de préférence le sel alcalin, dans un alcali en solution aqueuse, à des températures comprises entre 20 et 30 °C et à un pH de 11-12, avec un agent d'alkylation ou d'acylation, pour aboutir au sel correspondant du composé substitué en position 7, de formule générale A, et on isole celui-ci.

**5.** Procédé selon la revendication 4, caractérisé en ce que l'on effectue la nitrosation avec un nitrite alcalin, dans une suspension aqueuse acidifiée par de l'acide acétique, à des températures comprises entre +5 et -10 °C, et on isole le composé nitroso.

**6.** Procédé selon la revendication 4, caractérisé en ce que l'on effectue la sulfonation avec un hydrogénosulfite alcalin ou un disulfite alcalin en phase aqueuse, dans un intervalle de pH de 5 à 7, et on réduit sans isolement intermédiaire le composé d'addition bisulfite obtenu en l'acide aminosulfonique correspondant, dans une solution aqueuse acidifié par un acide minéral, à des températures comprises entre 20 et 60 °C.

**7.** Procédé selon la revendication 4, caractérisé en ce que l'on effectue l'oxydation avec une solution aqueuse d'acide nitrique à 15-25 % en poids, à des températures comprises entre 15 et 25 °C, et l'acide naphtoquinonesulfonique résultant est séparé sous forme de sel d'ammonium ou de potassium et isolé.

**8.** Procédé selon la revendication 4, caractérisé en ce que l'on fait réagir le sel d'ammonium ou de potassium de l'acide naphtoquinonesulfonique avec du p-toluènesulfonhydrazide en phase aqueuse ou organique, à des températures comprises entre 20 et 70 °C, pour aboutir aux sels correspondants de l'acide 7-hydroxy-1,2-naphtoquinone-(2)-diazide-4-sulfonique, et on isole celui-ci.

**9.** Procédé selon la revendication 4, caractérisé en que l'on effectue l'alkylation avec des esters aliphatiques ou aromatiques d'acides sulfoniques, ou avec des halogénures d'alkyle, d'aralkyle ou d'époxyalkyle.

**10.** Procédé selon la revendication 4, caractérisé en ce que l'on effectue l'acylation avec un halogénure de sulfonyle ou de carbonyle aromatique.

**11.** Procédé selon la revendication 9, caractérisé en ce que l'on utilise comme agent d'alkylation un sulfate de dialkyle.

**12.** Procédé selon la revendication 9, caractérisé en ce que l'on utilise comme agent d'alkylation du bromure d'époxypropyle ou du bromure de benzyle.

**13.** Procédé selon la revendication 10, caractérisé en ce que l'on utilise comme agent d'acylation du chlorure de benzoyle ou p-toluènesulfonyle.

**14.** Procédé selon la revendication 4, dans lequel

R représente un groupe alkyle ayant de 1 à 6 atomes de carbone, un groupe époxyalkyle ayant de 3 à 6 atomes de carbone, un groupe alkylcarbonyle ayant de 2 à 6 atomes de carbone ou un groupe alkylsulfonyle ayant de 1 à 4 atomes de carbone, dont les chaînes carbonées peuvent être interrompues par au moins un atome d'oxygène, un groupe aralkyle ayant de 7 à 10 atomes de carbone, un groupe arylcarbonyle ayant de 7 à 10 atomes de carbone, un groupe arylsulfonyle ayant de 6 à 10 atomes de carbone, qui peuvent être substitués par des groupes alkyle, trihalogénoalkyle, alcoxy, alkylcarbonyle ou alkylsulfonyle, ou par des halogènes, et

X représente un métal alcalin ou alcalino-terreux.

**15.** Procédé selon la revendication 14, dans lequel

X représente le sodium ou le potassium.

**16.** Utilisation des composés de formule générale A selon la revendication 1, en tant que produits intermédiaires pour la préparation de dérivés photosensibles dans des compositions et matériaux photosensibles.